(19) Europäisches Patentamt
European Patent Office
Office européen des brevets

(11) Veröffentlichungsnummer: **0 181 290 B1**

(12) **EUROPÄISCHE PATENTSCHRIFT**

(45) Veröffentlichungstag der Patentschrift: **18.09.91**

(21) Anmeldenummer: **85810506.7**

(22) Anmeldetag: **01.11.85**

(51) Int. Cl.$^5$: **C07D 487/04**, C09B 57/00, C08K 5/34, //(C07D487/04, 209:00,209:00)

Die Akte enthält technische Angaben, die nach dem Eingang der Anmeldung eingereicht wurden und die nicht in dieser Patentschrift enthalten sind.

(54) **Pigmentmischungen.**

(30) Priorität: **07.11.84 CH 5335/84**

(43) Veröffentlichungstag der Anmeldung: **14.05.86 Patentblatt 86/20**

(45) Bekanntmachung des Hinweises auf die Patenterteilung: **18.09.91 Patentblatt 91/38**

(84) Benannte Vertragsstaaten: **BE CH DE FR GB IT LI NL SE**

(56) Entgegenhaltungen:
**EP-A- 0 042 816**
**EP-A- 0 061 426**
**EP-A- 0 094 911**
**EP-A- 0 098 808**
**EP-A- 0 133 156**
**EP-A- 0 184 982**
**DE-A- 2 355 694**

**JOURNAL OF THE CHEMICAL SOCIETY, PERKIN TRANSACTIONS I, Nr. 1, 1976, Seiten 5-8, London, GB; J.M. SPRAKE et al.: "Addition products of 2-(N-Arylformimidoyl)pyridines and carbanions, and their reduction to octahydroindolizine derivatives"**

**CHEMICAL ABSTRACTS, Band 82, Nr. 1, 6. Januar 1975, Seite 359, Zusammenfassungsnr. 4148v, Columbus, Ohio, US; D.G. FARNUM et al.: "Attempted Reformatskii reaction of benzonitrile. 1,4-Dioxo-3,6-diphenylpyrrolo(3,4-c)pyrrole, a lactam analog of pentalene"**

(73) Patentinhaber: **CIBA-GEIGY AG**
**Klybeckstrasse 141**
**CH-4002 Basel(CH)**

(72) Erfinder: **Iqbal, Abul, Dr.**
**Im Schaiengarten 1**
**CH-4107 Ettingen(CH)**
Erfinder: **Pfenninger, Johannes, Dr.**
**Route du Confin 23**
**CH-1723 Marly(CH)**
Erfinder: **Rochat, Alain Claude, Dr.**
**Route de Schiffenen 38**
**CH-1700 Fribourg(CH)**
Erfinder: **Bäbler, Fridolin, Dr.**
**Route du Couchant 12**
**CH-1723 Marly(CH)**

**EP 0 181 290 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft Pigmentgemische aus verschiedenen 1,4-Diketopyrrolo-[3,4-c]-pyrrolen, die durch Umsetzung von 1 Mol Bernsteinsäurediester mit 2 Mol eines Gemisches mindestens zweier Nitrile, wobei eines der Nitrile in grossem Ueberschuss vorhanden ist, erhältlich sind. Aus der US-PS 4 579 949 ist die Umsetzung von 1 Mol Bernsteinsäurediester entweder mit 2 Mol eines Nitrils oder mit je 1 Mol zweier verschiedener Nitrile bekannt. Die so erhaltenen Produkte sind in verschiedener Hinsicht nicht immer befriedigend. Es ist nun gefunden worden, dass bei der Umsetzung mit einem Gemisch aus mindestes zwei Nitrilen in verschiedenem Molverhältnis zueinander Produkte mit verblüffend besseren Pigmenteigenschaften erhalten werden.

Die Erfindung betrifft demnach eine Pigmentmischung enthaltend mindestens zwei unterschiedliche 1,4-Diketopyrrolo-[3,4-c]-pyrrol-Pigmente erhältlich durch Umsetzung von
1 Mol eines Bernsteinsäurediesters mit 1,75-1,998 Mol eines Nitrils der Formel ACN und 0,002-0,25 Mol eines Nitrils der Formel BCN oder eines Gemisches mehrerer Nitrile der Formel BCN, deren Reste B sich von A unterscheiden, in einem organischen Lösungsmittel in Gegenwart einer starken Base bei erhöhter Temperatur und anschliessende Hydrolyse des entstandenen Reaktionsproduktes, wobei die Reste A und B jeweils Reste der Formeln

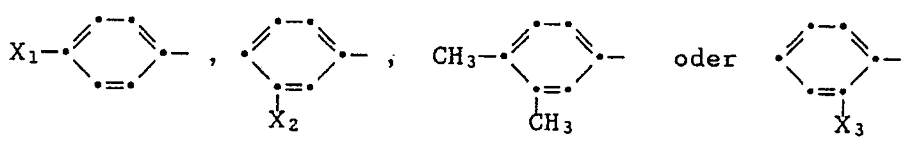

bedeuten, worin $X_1$ Wasserstoff, Methyl, Isobutyl, tert.-Butyl, Phenyl, Chlor, Brom, Methoxy, Phenoxy oder Cyan, $X_2$ Methyl, Chlor oder Cyan und $X_3$ Methyl oder Chlor darstellen oder A oder B $\beta$- oder $\gamma$-Pyridylreste bedeuten.

Besonders bevorzugt als Ausgangsstoffe sind Nitrile, worin A den Phenyl-, 4-Chlorphenyl- oder den 4-Biphenylyl-Rest und B einen Phenyl-, 3- oder 4-Chlorphenyl-, 4-Methylphenyl-, 4-Isobutylphenyl-, 4-Biphenylyl- oder 3-Cyanphenyl-Rest bedeuten. Als Beispiele seien die folgenden Nitrile genannt: Benzonitril, o-, m- oder p-Chlorbenzonitril, o-, m- oder p-Methylbenzonitril, p-tert.-Butylbenzonitril, p-Phenylbenzonitril, p-Methoxybenzonitril, p-Phenoxybenzonitril oder 3,4-Dimethylbenzonitril.

Man verwendet zweckmässig 1,8-1,99, insbesondere 1,9-1,98 Mol des Nitrils der Formel ACN und 0,01-0,2, insbesonders 0,02-0,1 Mol des Nitrils der Formel BCN oder einer Mischung mehrerer von ACN verschiedener Nitrile.

Die Nitrile ACN und BCN können sich sowohl durch die Art des Ringes oder die Art und/oder Stellung allfälliger Substituenten unterscheiden.

Bei den zu verwendenden Bernsteinsäurediestern kann es sich um Dialkyl-, Diaryl- oder Monoalkylmonoarylester handeln, wobei auch die Bernsteinsäuredialkyl- und -diarylester unsymmetrisch sein können. Bevorzugt verwendet man aber symmetrische Bernsteinsäurediester, insbesondere symmetrische Bernsteinsäuredialkylester. Liegt ein Bernsteinsäurediaryl- oder -monoaryl-monoalkylester vor, so bedeutet Aryl insbesondere unsubstituiertes oder durch Halogen, wie Chlor, $C_1$-$C_6$-Alkyl, wie Methyl, Aethyl, Isopropyl oder tert.-Butyl, oder $C_1$-$C_6$-Alkoxy, wie Methoxy oder Aethoxy substituiertes Phenyl. Aryl bedeutet bevorzugt unsubstituiertes Phenyl. Handelt es sich um einen Bernsteinsäuredialkyl- oder -monoalkyl-monoarylester, so kann Alkyl unverzweigt oder verzweigt sein, bevorzugt verzweigt, und vorzugsweise 1 bis 18, insbesondere 1 bis 12, vor allem 1 bis 8 und besonders bevorzugt 1 bis 5 C-Atome enthalten. Verzweigtes Alkyl ist bevorzugt sek.- oder tert.-Alkyl, wie z.B. sek.-Butyl, tert.-Butyl oder tert.-Amyl.

Beispiele für Bernsteinsäurediester sind Bernsteinsäure-dimethylester, -diäthylester, -dipropylester, -dibutylester, -dipentylester, -dihexylester, -diheptylester, -dioctylester, -diisopropylester, -di-sec.-butylester, -di-tert.-butylester, -di-tert.-amylester, -di-[1,1-dimethylbutyl]-ester, -di-[1,1,3,3-tetramethylbutyl]-ester, -di-[1,1-dimethylpentyl]-ester, -di-[1-methyl-1-äthyl-butyl]-ester, -di-[1,1-diäthylpropyl]-ester, -diphenylester, -di-(4-methylphenyl]-ester, -di-[2-methylphenyl]-ester, -di-[4-chlorphenyl]-ester, -monoäthyl-monophenylester.

Die Bernsteinsäure-diester und die Nitrile der Formel ACN und BCN sind bekannte Verbindungen und können nach bekannten Verfahren hergestellt werden.

Man führt die Umsetzung des Bernsteinsäurediesters mit den Nitrilen in einem organischen Lösungsmittel durch. Als Lösungsmittel eignen sich beispielsweise primäre, sekundäre oder tertiäre Alkohole mit 1 bis 10 C-Atomen, wie Methanol, Aethanol, n-Propanol, Isopropanol, n-Butanol, sek.-Butanol, tert.-Butanol, n-Pentanol, 2-Methyl-2-butanol, 2-Methyl-2-pentanol, 3-Methyl-3-pentanol, 2-Methyl-2-hexanol, 3-Aethyl-3-pentanol, oder 2,4,4-Trimethyl-2-pentanol, Glykole, wie Aethylenglykol oder Diäthylenglykol, ferner Aether, wie Tetrahydrofuran oder Dioxan, oder Glykoläther, wie Aethylenglykol-methyläther, Aethylenglykol-äthyläther, Diäthylenglykol-monomethyläther oder Diäthylenglykol-mono-äthyläther, ferner dipolar-aprotische Lösungsmittel, wie Aceto-nitril, Benzonitril, Dimethylformamid, N,N-Dimethylacetamid, Nitrobenzol oder N-Methylpyrrolidon, aliphatische oder aromatische Kohlenwasserstoffe, wie Benzol oder durch Alkyl, Alkoxy oder Halogen substituiertes Benzol, wie Toluol, Xylol, Anisol oder Chlorbenzol oder aromatische N-Heterocyclen, wie Pyridin, Picolin oder Chinolin. Zudem ist es auch möglich, das umzusetzende Nitril der Formel ACN bzw. BCN gleichzeitig als Lösungsmittel zu verwenden, falls es im Temperaturbereich flüssig ist, in dem die Umsetzung erfolgt. Die genannten Lösungsmittel können auch als Mischungen eingesetzt werden. Zweckmässigerweise verwendet man 5-20 Gew.-Teile Lösungsmittel auf 1 Gew.-Teil der Reaktionsteilnehmer. Man verwendet bevorzugt einen Alkohol als Lösungsmittel, insbesondere einen sekundären oder tertiären Alkohol. Bevorzugte tertiäre Alkohole sind tert.-Butanol und tert.-Amylalkohol.

Die Umsetzung wird in Gegenwart einer starken Base durchgeführt. Geeignete starke Basen sind z.B. Alkalihydroxide, wie Natrium-, Kalium- oder Lithiumhydroxid, oder Erdalkalihydroxide, wie Calcium- oder Magnesiumhydroxid, oder Alkaliamide, wie Lithium- oder Natriumamid, oder Alkalihydride, wie Lithiumhydrid oder Natriumhydrid, oder Erdalkali- oder Alkalialkoholate, die sich insbesondere von primären, sekundären oder tertiären aliphatischen Alkoholen mit 1 bis 10 C-Atomen ableiten, wie z.B. Natrium-, Kalium- oder Lithiummethylat, -äthylat, -n-propylat, -isopropylat, n-butylat, -sek.-butylat, -tert.-butylat, -2-methyl-2-butylat, -2-methyl-2-pentylat, -3-methyl-3-pentylat, -3-äthyl-3-pentylat, oder Erdalkali- oder Alkaliphenolate oder o-alkylsubstituierte -Phenolate wie Natrium-oder Kalium-o-Kresolat. Man kann aber auch ein Gemisch der genannten Basen verwenden.

Man verwendet als starke Base bevorzugt Alkalialkoholate, wobei Alkali insbesondere Natrium oder Kalium bedeutet, und das Alkoholat sich bevorzugt von einem sekundären oder tertiären Alkohol ableitet. Besonders bevorzugte starke Basen sind daher z.B. Natrium- oder Kalium-isopropylat, -sek.-butylat, -tert.-butylat und -tert.-amylat.

Man kann die starke Base in einer Menge von insbesondere 0,1 bis 4 Mol, bevorzugt 1,9 bis 2,2 Mol, bezogen auf den Reaktanden Bernsteinsäurediester einsetzen. Je nach Reaktionspartner und Verfahrensweise, wie z.B. bei Recyclisieren, können sich kleinere Basenmengen durchaus vorteilhaft auf die Ausbeute auswirken. In bestimmten Fällen kann andererseits auch ein Basenüberschuss die Ausbeute günstig beeinflussen. In der Regel genügen jedoch stöchiometrische Mengen an Base.

Die genannten starken Basen können zusammen mit einem Phasentransferkatalysator eingesetzt werden. Dies ist vor allem dann von Vorteil, wenn die Löslichkeit einer bestimmten Base in einem bestimmten Lösungsmittel gering ist. Die Phasentransferkatalysatoren können in einer Menge von 0,001 bis 50 Mol-%, vorzugsweise 0,01 bis 0,3 Mol-%, bezogen auf den Reaktanden Bernsteinsäurediester eingesetzt werden. Dazu eignen sich die üblichen, in der Literatur beschriebenen Phasentransferkatalysatoren, wie sie z.B. in CHEMTECH, Februar 1980, S. 111, Tabelle 1, aufgeführt sind, nämlich beispielsweise quaternäre Salze, cyclische Polyäther, offenkettige Polyäther, N-Alkylphosphoramide oder mit Methylen überbrückte Phosphor- oder Schwefeloxide.

Man arbeitet insbesondere bei einer Temperatur von 60 bis 140 °C, vorzugsweise 80 bis 120 °C.

Zur Umsetzung des Bernsteinsäurediesters mit den Nitrilen ist es grundsätzlich möglich, bei tieferer Temperatur alle Komponenten vorzulegen und dann das Gemisch in den Bereich der Reaktionstemperatur zu erwärmen, oder in beliebiger Reihenfolge die einzelnen Komponenten im Bereich der Reaktionstemperatur zueinander zuzugeben.

Eine bevorzugte Ausführungsform, die sich in der Regel besonders günstig auf die Ausbeute auswirkt, besteht darin, dass man das Nitril der Formel ACN mit BCN oder einem Gemisch mehrerer von ACN verschiedener Nitrile zusammen mit der Base vorlegt und den Bernsteinsäurediester im Bereich der Reaktionstemperatur zudosiert.

Insbesondere bei Bernsteinsäurediestern mit niederen Alkylresten und bei Alkoholaten, die sich aus niederen Alkoholen ableiten, wie z.B. Methanol, Aethanol, n-Propanol, Isopropanol oder tert.-Butanol, kann es sich als notwendig erweisen, den bei der Umsetzung entstehenden niederen Alkohol laufend aus dem Reaktionsmilieu zu entfernen, um höhere Ausbeuten zu erzielen.

Verwendet man als Lösungsmittel einen Alkohol und als Base ein Alkoholat, so kann es vorteilhaft sein, einen Alkohol und ein Alkoholat mit gleichen Alkylteilen zu wählen. Ebenso vorteilhaft kann es sein, wenn zudem noch der Bernsteinsäurediester ebensolche Alkylgruppen enthält.

Zur Hydrolyse des Kondensationsproduktes kann man eine Säure, einen Alkohol mit 1 bis 4 C-Atomen, wie Methanol oder Aethanol, vorzugsweise aber Wasser, verwenden. Als Säuren kommen z.B. aliphatische oder aromatische Carbon- oder Sulfonsäuren in Betracht, wie beispielsweise Ameisensäure, Essigsäure,

Propionsäure, Oxalsäure, Benzoesäure oder Benzolsulfonsäure. Weiterhin kommen als Säuren auch Mineralsäuren in Betracht, wie Chlorwasserstoff, dessen wässerige Lösung, sowie Kohlensäure, verdünnte Schwefel- und Phosphorsäure.

Bei der Hydrolyse fällt das Pigmentgemisch aus und kann durch Abfiltrieren isoliert werden.

Zur zusätzlichen Verbesserung der Pigmentformen können die erfindungsgemässen Pigmentmischungen nach der Hydrolyse im Reaktionsgemisch oder nach der Pigmentisolierung nachträglich in Wasser oder einem organischen Lösungsmittel, gegebenenfalls unter Druck, nachbehandelt werden. Vorzugsweise verwendet man solche organische Lösungsmittel, die über 80 °C sieden. Als besonders geeignet erweisen sich durch Halogenatome, Alkyl- oder Nitrogruppen substituierte Benzole, wie Xylole, Chlorbenzol, o-Dichlorbenzol oder Nitrobenzol, sowie Pyridinbasen, wie Pyridin, Picolin oder Chinolin, ferner Ketone, wie Cyclohexanon, Aether, wie Aethylenglykolmonomethyl- oder -monoäthyläther, Amide, wie Dimethylformamid oder N-Methylpyrrolidon, sowie Dimethylsulfoxid oder Sulfolan. Man kann die Nachbehandlung auch in Wasser in Gegenwart von organischen Lösungsmitteln und/oder mit Zusatz von oberflächenaktiven Substanzen durchführen.

Besonders transparente Pigmentformen erzielt man, wenn die erhaltenen Pigmentgemische einer nachträglichen Zerkleinerung, wie wässerige Nassmahlung, unterworfen werden.

Die erhaltene Pigmentmischung ist neu und kann zum Pigmentieren von hochmolekularem organischem Material verwendet werden, beispielsweise Celluloseäthern und -estern, wie Aethylcellulose, Nitro-cellulose, Celluloseacetat, Cellulosebutyrat, natürlichen Harzen oder Kunstharzen, wie Polymerisationsharze oder Kondensationsharze, Aminoplaste, insbesondere Harnstoff- und Melamin-Formaldehydharze, Alkydharze, Phenoplaste, Polycarbonate, Polyolefine, Polystyrol, Polyvinylchlorid, Polyamide, Polyurethane, Polyester, Gummi, Casein, Silikon und Silikonharze, einzeln oder in Mischungen.

Die erwähnten hochmolekularen organischen Verbindungen können einzeln oder in Gemischen als plastische Massen, Schmelzen oder in Form von Spinnlösungen, Lacken, Anstrichstoffen oder Druckfarben vorliegen. Je nach Verwendungszweck erweist es sich als vorteilhaft, die erfindungsgemäss zu verwendenden Pigmente als Toner oder in Form von Präparaten einzusetzen. Bezogen auf das zu pigmentierende hochmolekulare organische Material setzt man die Pigmentzusammensetzung in einer Menge von vorzugsweise 0,1 bis 10 Gew.% ein.

Die erfindungsgemässen Pigmentmischungen weisen gegenüber den aus US-Patent 4 415 685 bekannten einheitlichen Pigmenten verschiedene Vorteile auf:

- Sie lassen sich während der Synthese und/oder Konditionierung bezüglich der Partikelform und -grösse besser kontrollieren.
- Sie sind farbstärker und transparenter.
- Sie zeigen eine geringere Rekristallisationstendenz während Synthese und/oder Konditionierung.
- Sie weisen eine höhere Lagerstabilität in Lösungsmitteln, insbesondere in Lösungsmittel enthaltenden Lacken und Drucktinten auf.
- Sie zeichnen sich durch eine höhere Hitzestabilität in Kunststoffen aus, was sich in einer Nuancen- und Farbstärkenkonstanz bei verschiedenen Verarbeitungstemperaturen auswirkt.
- Wegen der höheren Transparenz und Farbstärke sind sis zur Massefärbung von Fasern besser geeignet.
- Sie können gegebenenfalls zu unerwarteten Nuancen mit interessanten Eigenschaften führen.

Die mit den vorliegenden Pigmentmischungen erhaltenen Färbungen, beispielsweise in Kunststoffen, Fasern, Lacken oder Drucken, zeichnen sich ausserdem durch gute Dispergierbarkeit, gute Ueberlackier-, Migrations-, Hitze-, Licht- und Wetterechtheit, sowie durch einen hohen Glanz aus.

Die folgenden Beispiele erläutern die Erfindung. Die Temperaturen sind in °C angegeben.

Beispiel 1:

27,6 g Natrium und 0,4 g Sulfobernsteinsäure-bis-2-äthylhexylester Natriumsalz werden in 480 ml tert.-Amylalkohol bei Rückflusstemperatur bis zur vollständigen Auflösung gerührt. Man kühlt auf 90° ab, fügt 104,6 g 4-Chlorbenzonitril und 4,1 g Benzonitril zu und dosiert anschliessend über 3 Stunden 81,2 g Bernsteinsäure-diisopropylester zu. Man lässt 1 Stunde bei Rückflusstemperatur ausreagieren und giesst das Reaktionsgemisch auf 800 ml Wasser. Die Hydrolysemischung wird während einer Stunde gekocht und anschliessend wird zur Entfernung des organischen Lösungsmittels während einer Stunde Wasserdampf eingeleitet. Die Pigmentsuspension wird filtriert, mit Wasser und Methanol gewaschen und im Vakuum bei 80° getrocknet. Man erhält 106,3 g Pigment, das, in PVC eingearbeitet, eine rote Färbung ergibt.

| C, H, N-Analyse: | : C | H | N |
|---|---|---|---|
| Gef. | 61,04 | 3,01 | 7,79 |

Beispiel 2:

6,9 g Natrium und 0,1 g Sulfobernsteinsäure-bis-2-äthylhexylester Natriumsalz werden in 120 ml tert.-Amylalkohol bei Rückflusstemperatur bis zur vollständigen Auflösung gerührt. Man kühlt auf 90° ab, fügt 21,6 g 4-Chlorbenzonitril und 1,6 g 4-tert.-Butylbenzonitril zu und dosiert anschliessend über 3 Stunden 20,3 g Bernsteinsäure-diisopropylester zu. Man lässt 1 Stunde bei Rückflusstemperatur kochen und giesst das Reaktionsgemisch auf 200 ml Wasser. Das hydrolysierte Produkt wird wie in Beispiel 1 aufgearbeitet. Man erhält 26,3 g Pigment, das, in PVC eingearbeitet, eine rote Färbung ergibt.

| C, H, N-Analyse: | C | H | N |
|---|---|---|---|
| Gef. | 61,00 | 3,19 | 7,71 |

Beispiel 3:

Man geht gleich vor wie in Beispiel 2, setzt aber ein Nitrilgemisch aus 30,2 g 4-tert.-Butylbenzonitril und 1,4 g 3-Chlorbenzonitril ein und führt die Kondensation bei 105° durch. Man isoliert 21,1 g Pigment, das in PVC eingearbeitet, eine rot-orange Färbung ergibt.

Beispiel 4:

In einem 1,5 1 Glasbehälter werden 500 ml tert.-Amylalkohol vorgelegt und langsam Stickstoff eingsleitet. 27,6 g Natrium, anschliessend als Emulgator 0,4 g Sulfobernsteinsäure-bis-2-äthylhexylester-Na-Salz werden in den tert.-Amylalkohol gegeben und das Gemisch langsam auf 95-102° erwärmt. Unter heftigem Rühren wird das geschmolzene Matall im Alkohol aufgelöst. Die erhaltene Lösung wird auf ca. 80° abgekühlt und mit 81,6 g Benzonitril, sowie 1,1 g p-Chlorbenzonitril versetzt. Das Gemisch wird auf 110° erwärmt, unter Stickstoffeinleitung und Rühren lässt man langsam innert 3 Stunden 80,8 g Bernsteinsäure-diisopropylester zutropfen und gleichzeitig den sich bildenden Isopropanol abdestillieren. Die Temperatur sinkt gegen Ende der Reaktion auf 104°. Die erhaltene Pigmentsuspension lässt man 2 Stunden ausreagieren, gleichzeitig destilliert man etwas Isopropanol/tert.-Amylalkohol ab. In einem Becherglas werden 700 ml Wasser (15-25°) vorgelegt und unter gutem Rühren die Pigmentsuspension während ca. 3 Minuten eingetragen. Das sich gebildete Zweiphasengemisch wird während einer Stunde gerührt, in ein mit einem Kühler ausgerüsteten Glasgefäss umgegossen, auf Rückflusstemperatur erwärmt und während 6 Stunden am Rückfluss gerührt, dann das Lösungsmittel durch das Einleiten von Wasserdampf abdestilliert. Die erhaltene wässerige Pigmentsuspension wird heiss abfiltriert, mit heissem Wasser gewaschen, der Presskuchen im Vakuumtrockenschrank bei 80° getrocknet und pulverisiert. Man erhält 70 g rotes Pigmentpulver, das, in PVC eingearbeitet, eine rote Färbung ergibt.

Beispiel 5:

Verfährt man analog wie in Beispiel 4, verwendet aber anstelle von 81,6 g 80,0 g Benzonitril und anstelle von 1,1 g 3,4 g p-Chlorbenzonitril, so erhält man eine Pigmentzusammensetzung mit gleich guten Eigenschaften wie im Beispiel 4.

Beispiel 6-12:

Man geht gleich vor wie in Beispiel 2, setzt jedoch die in nachstehender Tabelle als ACN und BCN aufgeführten Nitrile ein.

| Bsp. Nr. | ACN | BCN |
|---|---|---|
| 6 | 27,2 g 4-Chlorbenzonitril | 0,4 g 4-Cyan-biphenyl |
| 7 | 26,7 g " | 1,1 g " |
| 8 | 26,1 g " | 1,8 g " |
| 9 | 24,8 g " | 3,6 g " |
| 10 | 26,7 g " | 0,6 g 4-Cyan-pyridin |
| 11 | 26,1 g " | 1,1 g " |
| 12 | 24,8 g " | 2,1 g " |

Beispiel 13-18:

Man geht gleich vor wie in Beispiel 2, setzt jedoch die in nachstehender Tabelle als ACN und BCN aufgeführten Nitrile ein.

| Bsp. Nr. | ACN | BCN |
|---|---|---|
| 13 | 34,3 g 4-Cyan-biphenyl | 1,0 g Benzonitril |
| 14 | 32,5 g " | 2,1 g " |
| 15 | 34,3 g " | 1,4 g 4-Chlor-benzonitril |
| 16 | 32,5 g " | 2,8 g " |
| 17 | 34,3 g " | 1,6 g 4-t-Butyl-benzonitril |
| 18 | 32,5 g " | 3,2 g " |

Beispiel 19:

Eine Mischung von 130 g Steatitkugeln von 8 mm Durchmesser, 47,5 g Alkydmelamineinbrennlack, bestehend aus 60 g Beckosol 27-320® (Reichhold Chemie AG), 60 % in Xylol, 36 g Super Beckamin 13-501® (Reichhold Chemie AG), 50 % in einer Mischung von 2 g Xylol und 2 g Aethylenglykolmonomethyläther, und 2,5 g der nach den Beispielen 4 respektiv 5 erhaltenen Pigmentzusammensetzungen werden in einer Glasflasche mit Twist-off-Verschluss während 120 Stunden auf dem Rollgestell dispergiert. Nach Abtrennen der Steatitkugeln wird der Farblack auf Weisskarton appliziert und anschliessend während 30 Minuten bei 130° eingebrannt. (Lackschichtdicke ca. 50 µm).

Der Farbton der auf diese Art eingefärbten Lacke ist nach DIN 6174 wie folgt charakterisiert:

| | L* | A* | B* |
|---|---|---|---|
| Pigmentmischung gemäss Beispiel 4 | 45,20 | 51,23 | 29,86 |
| Pigmentmischung gemäss Beispiel 5 | 44,30 | 50,13 | 27,97 |

Beispiel 20:

0,6 g der gemäss Beispiel 1 erhaltenen Pigmentmischung werden mit 67 g Polyvinylchlorid, 33 g Dioctylphthalat, 2 g Dibutylzinndilaurat und 2 g Titandioxid zusammengemischt und auf dem Walzenstuhl während 15 Minuten bei 160° zu einer dünnen Folie verarbeitet. Die so erzeugte rote PVC-Folie ist farbstark, migrations- und lichtbeständig.

Beispiel 21:

Eine Mischung von 1,0 g der nach Beispiel 4 erhaltenen Pigmentmischung, 1,0 g Antioxydant IRGANOX IOIO® (Handelsname der CIBA-GEIGY AG) und 1000,0 g Polyäthylen-HD Granulat VESTOLEN A-60-16® (Handelsname der Firma HUELS) wird während 15 Minuten in einer 3 l Glasflasche auf dem Röhnradmischer vorgemischt. Danach wird die Mischung in zwei Passagen auf einem Einwellenextruder extrudiert, das so erhaltene Granulat auf der Spritzgussmaschine (Allround Aarburg 200®) bei 220° zu Platten verspritzt und 5 Minuten bei 180° nachgepresst. Die Pressplatten weisen farbstarke rote Färbungen mit ausgezeichneten Echtheiten auf.

Beispiel 22:

Verfährt man analog wie in Beispiel 21, spritzt aber das eingefärbte Granulat bei 270° anstelle von 220°, so erhält man analog gefärbte Pressplatten.

Beispiel 23:

1000 g Polypropylengranulat, DAPLEN PT-55®, (Handelsname der Firma Chemie LINZ) und 20 g eines 50%-igen Pigmentpräparates, bestehend aus 10 g der nach Beispiel 4 erhaltenen Pigmentzusammensetzung und 10 g Mg-Behenat, werden in einer Mischtrommel intensiv vermischt. Das so behandelte Granulat wird bei 260 bis 285° nach dem Schmelzspinnverfahren versponnen. Man erhält rotgefärbte Fasern mit sehr guten Licht- und textilen Echtheiten.

**Patentansprüche**

1. Pigmentmischung enthaltend mindestens zwei unterschiedliche 1,4-Diketopyrrolo-[3,4-c]-pyrrol-Pigmente erhältlich durch Umsetzung von

   1 Mol eines Bernsteinsäurediesters mit 1,75-1,998 Mol eines Nitrils der Formel ACN und 0,002-0,25 Mol eines Nitrils der Formel BCN oder eines Gemisches mehrerer Nitrile der Formel BCN, deren Reste B sich von A unterscheiden, in einem organischen Lösungsmittel in Gegenwart einer starken Base bei erhöhter Temperatur und anschliessende Hydrolyse des entstandenen Reaktionsproduktes, wobei die Reste A und B jeweils Reste der Formeln

   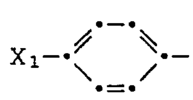 , 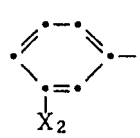 , 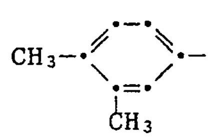 oder 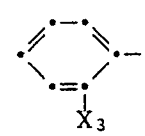

   bedeuten, worin $X_1$ Wasserstoff, Methyl, Isobutyl, tert.-Butyl, Phenyl, Chlor, Brom, Methoxy, Phenoxy oder Cyan, $X_2$ Methyl, Chlor oder Cyan und $X_3$ Methyl oder Chlor darstellen oder A oder B $\beta$- oder $\gamma$-Pyridylreste bedeuten.

2. Pigmentmischung gemäss Anspruch 1, dadurch gekennzeichnet, dass man auf 1 Mol eines Bernsteinsäurediesters 1,8-1,99 Mol des Nitrils der Formel ACN und je 0,01-0,2 Mol des Nitrils BCN oder eines Gemisches mehrerer Nitrile der Formel BCN, deren Reste B sich von A unterscheiden, verwendet.

3. Pigmentmischung gemäss Anspruch 1, dadurch gekennzeichnet, dass man auf 1 Mol eines Bernsteinsäurediesters 1,9-1,98 Mol des Nitrils der Formel ACN und je 0,02-0,1 Mol des Nitriles BCN oder eines Gemisches mehrerer Nitrile der Formel BCN, deren Reste B sich von A unterscheiden, verwendet.

4. Pigmentmischung gemäss Anspruch 1, dadurch gekennzeichnet, dass man ein einheitliches Nitril BCN verwendet.

5. Pigmentmischung gemäss Anspruch 1, dadurch gekennzeichnet, dass man von Nitrilen ausgeht, worin A den Phenyl-, 4-Chlorphenyl-, oder den 4-Biphenylyl-Rest und B den Phenyl-, 3- oder 4-Chlorphenyl-, 4-Methylphenyl-, 4-Isobutylphenyl-, 4-Bisphenylyl- oder 3-Cyanphenyl-Rest bedeuten.

6. Verfahren zum Pigmentieren von hochmolekularem organischem Material, gekennzeichnet durch die

Verwendung einer Pigmentmischung gemäss Anspruch 1.

7. Hochmolekulares organisches Material enthaltend eine Pigmentmischung gemäss Anspruch 1.

## Claims

1. A mixture of pigments comprising at least two different 1,4-diketopyrrolo[3,4-c]pyrrole pigments obtainable by reacting 1 mole of a succinic acid diester with 1.75 to 1.998 moles of a nitrile of the formula ACN and 0.002 to 0.25 mole of a nitrile of the formula BCN or of a mixture of several nitriles of the formula BCN, the radicals B of which are different from A, in an organic solvent in the presence of a strong base at elevated temperature, and subsequently hydrolysing the resultant reaction product, the radicals A and B being in each case radicals of the formula

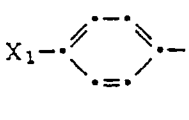 , 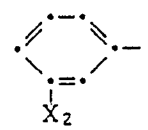 , 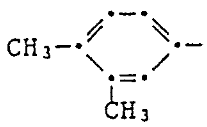 or 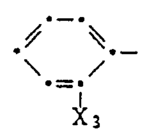

in which $X_1$ is hydrogen, methyl, isobutyl, tert-butyl, phenyl, chlorine, bromine, methoxy, phenoxy or cyano, $X_2$ is methyl, chlorine or cyano and $X_3$ is methyl or chlorine, or A or B are $\beta$-pyridyl or $\gamma$-pyridyl radicals.

2. A mixture of pigments according to claim 1, wherein 1.8 to 1.99 moles of the nitrile of the formula ACN and 0.01 to 0.2 mole of the nitrile BCN or of a mixture of several nitriles of the formula BCN, the radicals B of which are different from A, are used per mole of a succinic acid diester.

3. A mixture of pigments according to claim 1, wherein 1.9 to 1.98 moles of the nitrile of the formula ACN and 0.02 to 0.1 mole of the nitrile BCN or of a mixture of several nitriles of the formula BCN, the radicals B of which are different from A, are used per mole of a succinic acid diester.

4. A mixture of pigments according to claim 1, wherein a uniform nitrile BCN is used.

5. A mixture of pigments according to claim 1, wherein the starting materials are nitriles in which A is the phenyl, 4-chlorophenyl or 4-biphenylyl radical and B is the phenyl, 3-chlorophenyl, 4-chlorophenyl, 4-methylphenyl, 4-isobutylphenyl, 4-biphenylyl or 3-cyanophenyl radical.

6. A method of pigmenting organic material of high molecular weight, which comprises using a mixture of pigments according to claim 1.

7. Organic material of high molecular weight comprising a mixture of pigments according to claim 1.

## Revendications

1. Mélange de pigments contenant au moins deux pigments de type bioxo-1,4 pyrrolo[3,4-c]pyrrole différents, que l'on peut obtenir en faisant réagir :

1 mole d'un diester de l'acide succinique avec 1,75 à 1,998 mole d'un nitrile de formule ACN et 0,002 à 0,25 mole d'un nitrile de formule BCN ou d'un mélange de plusieurs nitriles de formule BCN, dont les restes B diffèrent de A, dans un solvant organique en présence d'une base forte, à température élevée, et en effectuant une hydrolyse du produit résultant de la réaction, les restes A et B représentant à chaque fois des restes de formule :

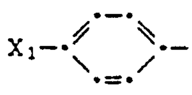 , 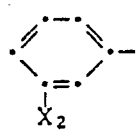 , 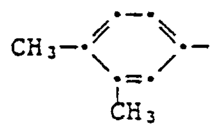 ou 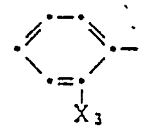

formules dans lesquelles $X_1$ représente un atome d'hydrogène ou un groupe méthyle, isobutyle, tertiobutyle, phényle, chloro, bromo, méthoxy, phénoxy ou cyano ; $X_2$ représente un groupe méthyle, chloro ou cyano, et $X_3$ représente un groupe méthyle ou chloro, ou bien A ou B représente des restes bêta-, ou gamma-pyridyles.

**2.** Mélange de pigments selon la revendication 1, caractérisé en ce que, pour 1 mole d'un diester de l'acide succinique, on utilise 1,8 à 1,99 mole du nitrile de formule ACN et 0,01 à 0,2 mole du nitrile BCN ou d'un mélange de plusieurs nitriles de formule BCN, dont les restes B diffèrent de A.

**3.** Mélange de pigments selon la revendication 1, caractérisé en ce que, pour 1 mole d'un diester de l'acide succinique, on utilise 1,9 à 1,98 mole du nitrile de formule ACN et 0,02 à 0,1 mole du nitrile BCN ou d'un mélange de plusieurs nitriles de formule BCN, dont les restes B diffèrent de A.

**4.** Mélange de pigments selon la revendication 1, caractérisé en ce qu'on utilise un nitrile BCN homogène.

**5.** Mélange de pigments selon la revendication 1, caractérisé en ce qu'on part de nitriles dans lesquels A représente le reste phényle, chloro-4 phényle ou 4-biphénylyle,et B représente le reste phényle, chloro-3 ou chloro-4 phényle, méthyl-4 phényle, isobutyl-4 phényle, bisphénylyle-4 ou cyano-3 phényle.

**6.** Procédé pour pigmenter de la matière organique à poids moléculaire élevé, caractérisé par l'utilisation d'un mélange de pigments selon la revendication 1.

**7.** Matière organique à poids moléculaire élevé, contenant un mélange de pigments selon la revendication 1.